# FASCICULE DE BREVET EUROPEEN

(11) **EP 2 821 393 B1**
(45) Date de publication et mention de la délivrance du brevet: **06.04.2016**
(21) Numéro de dépôt: 14174528.1
(22) Date de dépôt: 26.06.2014
(51) Int. Cl.: C07C 255/65, C07C 245/04, C07C 253/30

(54) **Procédé de synthèse de composés azo**
Syntheseverfahren von Stickstoffverbindungen
Method for the synthesis of azo compounds

(30) Priorité: 02.07.2013 FR 1356424
(43) Date de publication de la demande: 07.01.2015
(73) Titulaire: ARKEMA FRANCE, 92700 Colombes (FR)
(72) Inventeur: Sage, Jean-Marc, 69600 OULLINS (FR); Bossoutrot, Jean-Michel, 69630 CHAPONOST (FR)

(56) Documents cités:
- WO-A1-2006/067315
- US-A- 4 637 868
- DATABASE CA [Online] CHEMICAL ABSTRACTS SERVICE, COLUMBUS, OHIO, US; MACHO, VENDELIN ET AL: "2-2'-Azobisisobutyronitrile", XP002718465, extrait de STN Database accession no. 1987:19168 & CS 232 350 B1 (CZECH.) 16 janvier 1985 (1985-01-16)
- EYAL DRUG ET AL: "Catalytic oxidation of hydrazo derivatives promoted by a TiCl3/HBr system", JOURNAL OF THE AMERICAN CHEMICAL SOCIETY., vol. 129, 2007, pages 13784-13785, XP002718466, USAMERICAN CHEMICAL SOCIETY, WASHINGTON, DC. ISSN: 0002-7863

## Description

### DOMAINE TECHNIQUE

La présente invention se rapporte à un procédé de synthèse d'un composé azo, tel que l'azo-bis-isobutyronitrile (AIBN ou AZDN), par oxydation d'un composé hydrazo à l'aide de peroxyde d'hydrogène. Ce procédé comprend une étape d'ajout au milieu réactionnel d'un agent réducteur particulier, tel que l'hydrazine.

### ARRIÈRE-PLAN DE L'INVENTION

L'azo-bis-isobutyronitrile (ou AIBN) est un composé azoïque couramment utilisé dans les procédés de polymérisation radicalaire, en tant qu'amorceur ou catalyseur. Il est également connu comme agent gonflant pour la fabrication de mousses en PVC ou de joints en silicone.

L'AIBN est classiquement produit en convertissant la cyanohydrine d'acétone en hydrazine, suivie d'une oxydation au chlore gazeux (US-2,469,358 ; WO 2006/067315). Ce procédé a également été appliqué à d'autres composés azoïques tels que l'azodicarbonamide (GB 976552). Il a pour inconvénient majeur, outre la dangerosité intrinsèque du chlore, de générer de l'acide chlorhydrique comme sous-produit, de sorte que les effluents, produits par ailleurs en grande quantité, ne peuvent pas être facilement recyclés. On comprend que le procédé au chlore n'est donc pas adapté aux contraintes environnementales actuelles.

Pour surmonter cet inconvénient, il a été proposé des procédés de synthèse de composés azo n'utilisant pas de chlore, mais du peroxyde d'hydrogène en tant qu'agent oxydant. Ces procédés nécessitent la présence d'un agent activant, généralement un composé du brome tel qu'un bromure, ou encore un dérivé d'iode, utilisés en milieu acide, pour que la réaction soit suffisamment rapide et complète. De tels procédés ont notamment été décrits dans les documents US-4,637,868 ; RO 90707 ; RO 107406 ; RO 106881. La réactivité du peroxyde d'hydrogène, et ainsi le rendement de la réaction, sont généralement améliorés par l'emploi de catalyseurs métalliques en plus des composés de brome ou d'iode (DRUG et al., JACS 2007, 129(45), 13784-13785 ; JP52-144622 ; JP52-133924), en particulier de catalyseurs à base de molybdène ou de tungstène (CS 237123 ; CS 239407 ; CS 232350 ; PALOMO et al., Afinidad, 1985, 42(397), 312-314) qui ont l'avantage d'être moins toxiques que le tellure, le vanadium ou le sélénium, par exemple.

En dépit de ses avantages incontestables par rapport au procédé au chlore, le procédé au peroxyde d'hydrogène génère toutefois lui aussi des effluents potentiellement dommageables pour l'environnement, à savoir les eaux-mères résultant de la filtration du milieu réactionnel pour en séparer l'AIBN. Ces eaux-mères contiennent en effet une quantité non négligeable du catalyseur et des activateurs utilisés. Il a donc été suggéré, non seulement pour des questions environnementales, mais également pour améliorer l'économie du procédé, de recycler ces eaux-mères dans la réaction, éventuellement après concentration (CS 239407; CS 237123).

Malheureusement, il a été observé qu'en présence de certains catalyseurs, les eaux-mères n'étaient pas stables dans le temps et avaient tendance à précipiter, ce qui est préjudiciable à leur recyclage ou à leur traitement en vue d'un rejet dans l'environnement. La Demanderesse a mis en évidence que l'ajout de certains agents réducteurs au milieu réactionnel permettait de résoudre ce problème.

Il est ainsi proposé un procédé de synthèse de composés azo de pureté élevée, avec un rendement supérieur à 90%, permettant un traitement aisé des effluents en vue de leur recyclage dans le procédé et/ou de leur rejet dans l'environnement, de sorte que l'économie du procédé et son impact environnemental se trouvent améliorés.

### EXPOSE DE L'INVENTION

La présente invention a ainsi pour objet un procédé de synthèse d'un composé azo, comprenant les étapes successives consistant à :
a) faire réagir du peroxyde d'hydrogène avec une solution aqueuse d'un composé hydrazo renfermant au moins un acide organique ou inorganique, au moins un bromure de métal alcalin ou d'hydrogène et au moins un composé soluble dans l'eau choisi parmi les sels et acides à base d'un métal catalytique choisi parmi le molybdène et le tungstène, de manière à former une solution renfermant un composé azo,
b) ajouter à la solution obtenue à l'étape (a) au moins un agent réducteur choisi parmi l'hydrazine, le sulfite de sodium, le bisulfite de sodium et leurs mélanges,
c) récupérer tout ou partie du mélange réactionnel ainsi obtenu,
d) séparer le mélange réactionnel récupéré en une fraction contenant le composé azo et une fraction d'eaux-mères, et
e) éventuellement, laver la fraction contenant le composé azo pour l'isoler.

### DESCRIPTION DÉTAILLÉE DE MODES DE RÉALISATION

Le procédé selon l'invention comprend une première étape, ou étape (a), d'oxydation d'un composé hydrazo, en solution aqueuse, par du peroxyde d'hydrogène.

Le composé hydrazo peut être choisi parmi les composés hydrazo symétriques porteurs de fonctions azotées, en particulier de fonctions nitriles ou amines, telles que le 2,2'-hydrazo-bis-isobutyronitrile, le 2,2'-hydrazo-bis-méthylbutyronitrile, le 1,1'-hydrazo-bis-cyclohexanecarbonitrile ou le 2,2'-hydrazo-dicarbonamide, de préférence le 2,2'-hydrazo-bis-isobutyronitrile.

Outre le composé hydrazo, la solution aqueuse peut comprendre au moins un agent tensioactif, notamment un tensioactif non ionique, en particulier un sulfosuccinate d'alkyle tel que le sulfosuccinate de di-2-éthylhexyle.

Le pH de la solution aqueuse est acide et de préférence compris entre 0 et 2.

Le peroxyde d'hydrogène est généralement introduit dans la solution aqueuse à une température de 0 à 40°C, de préférence de 0 à 20°C, pendant une durée allant de 2 à 6 heures. Il est généralement utilisé en léger excès molaire par rapport au composé hydrazo. Le rapport molaire du peroxyde d'hydrogène au composé hydrazo est ainsi avantageusement compris entre 1:1 et 1,1:1, et de préférence entre 1,01:1 et 1;05:1, bornes incluses.

Cette réaction d'oxydation est mise en oeuvre en présence d'au moins un catalyseur, d'au moins un activateur et d'au moins un acide, également présents dans la solution aqueuse. L'ordre d'introduction des différents composés dans cette solution n'est pas critique, et on peut ainsi y ajouter les trois composés précités, puis le composé hydrazo et enfin le tensioactif éventuellement utilisé.

Le catalyseur comprend un composé soluble dans l'eau choisi parmi les sels et acides à base d'un métal catalytique choisi parmi le molybdène et le tungstène, de préférence le molybdène. Des exemples de tels composés solubles dans l'eau sont notamment : les sels de métal alcalin ou d'ammonium de molybdène, les sels de métal alcalin ou d'ammonium de tungstène, l'acide phosphomolybdique et ses sels alcalins ou d'ammonium, l'acide phosphotungstique et ses sels alcalins ou d'ammonium, les molybdosulfates et leurs mélanges. L'acide phosphomolybdique et ses sels alcalins ou d'ammonium, notamment le molybdate d'ammonium, sont préférés.

L'activateur est un bromure de métal alcalin ou d'hydrogène. Il peut être en particulier du bromure de sodium. Il peut être utilisé dans un rapport molaire de l'activateur au composé hydrazo allant par exemple de 1:2 à 1:10 voire de 1:4 à 1:8.

Comme acide, on peut choisir un acide organique ou inorganique quelconque. On préfère toutefois que l'acide inorganique soit choisi parmi les acides chlorhydrique, sulfurique, bromhydrique, phosphorique et leurs mélanges, de préférence l'acide chlorhydrique, et que l'acide organique soit choisi parmi les acides formique, acétique et leurs mélanges. Il peut être utilisé dans un rapport molaire de l'acide au composé hydrazo allant par exemple de 1:1 à 1:5.

On obtient ainsi une solution aqueuse renfermant un composé azo et une certaine quantité de métal catalytique et de peroxyde d'hydrogène, notamment. La seconde étape ou étape (b) du procédé selon l'invention comprend l'ajout, à cette solution, d'au moins un agent réducteur choisi parmi l'hydrazine, le sulfite de sodium, le bisulfite de sodium et leurs mélanges. Selon une forme d'exécution préférée de l'invention, l'agent réducteur est l'hydrazine dans le cas où le composé soluble dans l'eau est l'acide phosphomolybdique. L'hydrazine a l'avantage de ne former que de l'eau et de l'azote gazeux dans les effluents, et dans la boucle de recyclage du procédé, et ainsi de ne pas accumuler de sels au cours du recyclage. L'agent réducteur est généralement utilisé en quantité nécessaire et suffisante pour neutraliser l'excès de peroxyde d'hydrogène mesuré à l'issue de l'étape (a) du procédé.

On obtient ainsi un mélange réactionnel qui est ensuite récupéré en tout ou partie, par exemple à hauteur de 30 à 60% en poids, notamment de 45 à 55% en poids, dans la troisième étape, ou étape (c) du procédé selon l'invention.

Le mélange réactionnel éventuellement non récupéré peut être recyclé dans l'étape (a), tandis que le mélange réactionnel récupéré est séparé, dans une étape (d), en une fraction contenant le composé azo et une fraction d'eaux-mères. Le composé azo peut être séparé par filtration ou centrifugation, de préférence par centrifugation.

La fraction contenant le composé azo peut être lavée une ou plusieurs fois à l'eau, dans une étape (e), de façon à récupérer un composé azo ayant une pureté supérieure à 90% et des eaux de lavage.

Dans une étape ultérieure (f) du procédé, la fraction d'eaux-mères peut de son côté être recyclée en tout ou partie dans l'étape (a), étant entendu que les étapes (a) à (f) sont éventuellement répétées au moins une fois, c'est-à-dire que la fraction d'eaux-mères peut être recyclée au moins deux fois. Dans une variante du procédé selon l'invention, la fraction d'eaux-mères peut être concentrée, notamment par distillation, avant d'être recyclée. Le distillat obtenu peut alors être facilement traité, par exemple incinéré, en vue d'être rejeté dans l'environnement.

Comme indiqué précédemment, le procédé selon l'invention permet un traitement aisé des effluents en vue de leur recyclage dans le procédé et/ou de leur traitement en vue de leur rejet dans l'environnement. A cette fin, il peut comprendre une étape supplémentaire de traitement de tout ou partie de la fraction d'eaux-mères produites dans l'étape (d) et/ou des eaux de lavage produites dans l'étape (e) à l'aide d'un adsorbant, tel que du charbon actif, afin de retenir le métal catalytique. Le procédé selon l'invention peut en outre comprendre une étape de récupération du métal catalytique sous forme de solution aqueuse, par traitement de l'adsorbant à l'aide d'une solution aqueuse basique, notamment d'hydroxyde de sodium. On préfère industriellement faire passer la fraction d'eaux-mères et/ou des eaux de lavage à traiter sur une colonne contenant l'adsorbant, sous une forme par exemple granulée, puis récupérer le métal catalytique par passage d'une solution basique sur cette colonne, selon les techniques bien connues d'utilisation de ces adsorbants.

La solution aqueuse de métal catalytique peut ainsi être recyclée dans l'étape (a) du procédé, éventuellement après concentration, tandis que les eaux-mères résultant de la filtration de l'adsorbant et/ou les eaux de lavage de l'adsorbant peuvent être rejetées dans l'environnement. Il a été démontré dans les exemples ci-après que cette variante de l'invention permet de récupérer et recycler une quantité efficace de catalyseur, tout en rejetant dans l'environnement un effluent très pauvre en catalyseur.

Le procédé selon l'invention permet d'obtenir en un temps raisonnable une granulométrie élevée des composés azo produits, typiquement voisine de 150 µm (telle que mesurée par diffraction laser), qui peut s'avérer avantageuse dans certaines applications.

L'invention sera mieux comprise à la lumière des exemples non limitatifs suivants, qui sont donnés à titre uniquement illustratif et n'ont pas pour but de limiter la portée de l'invention, définie par les revendications annexées.

### EXEMPLES

### Réactifs

Dans les exemples suivants :
- DHC désigne l'hydrazo-bis-isobutyronitrile, composé hydrazo obtenu industriellement par réaction du cyanhydrine d'acétone sur l'hydrate d'hydrazine, filtration puis lavage à l'eau, et conservé au réfrigérateur (T<10°C). Son taux d'humidité est de 12,7% et sa pureté est supérieure à 99% par analyse.
- L'acide phosphomolybdique utilisé est un produit vendu par la société Aldrich qui correspond à la formule H₃[P(Mo₃O₁₀)₄].xH₂O et est utilisé comme tel (masse molaire 1825.25 g/mol sous forme anhydre). Le produit utilisé possède une teneur de 50% en molybdène.
- Le molybdate d'ammonium est obtenu via la société Aldrich et correspond à la formule H₂₄Mo₇N₆O₂₄.4H₂O de masse molaire 1235,86 g/mol.
- Le molybdate de sodium dihydraté est obtenu via la société Aldrich et correspond à la formule Na₂,MoO₄.2H₂O de masse molaire 241,95 g/mol.
- DOSS désigne le sulfosuccinate de di(2-éthylhexyle).
- AZDN (ou AIBN) désigne l'azo-bis-isobutyronitrile.

### Méthode de prélèvement et de dosage du taux de peroxyde résiduel

Dans les exemples suivants, les taux de peroxyde résiduel sont mesurés comme suit. On prélève environ 3-5 mL du mélange réactionnel en suspension que l'on filtre afin d'éliminer le DHC et l'AZDN solide présents. On pèse environ un gramme de la solution filtrée que l'on introduit dans un flacon de 250 mL, on rajoute 50 mL d'eau distillée, 15 mL d'acide sulfurique à 30% en poids et 15 mL d'une solution de KI à 30%. Le flacon est bouché puis laissé à l'obscurité pendant 15 mn. On titre ensuite avec une solution de thiosulfate de normalité 0,1 N jusqu'à disparition de la coloration jaune. La teneur en peroxyde d'hydrogène est calculée ainsi :

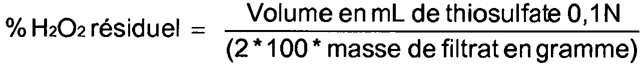

### Méthode de dosage du Molybdène résiduel.

Il est effectué par la technique d'ICP (ou Plasma Couplé Induction).

### Exemple 1 : Association de réducteurs avec l'acide phosphomolybdique

On utilise un réacteur en verre de 1 litre équipé d'une agitation mécanique de type ancre (vitesse d'agitation 500 tours/min). Le réacteur est muni d'un réfrigérant. L'introduction de la solution de peroxyde d'hydrogène est assurée au moyen d'une pompe péristaltique via un tube souple par le haut du réacteur. Le réacteur est de type double enveloppe, refroidi à l'aide d'un cryostat par une circulation d'eau froide dans la double enveloppe.

Le bain cryostat est réglé à 13°C et mis en circulation sur le réacteur. On introduit dans le réacteur 116,8 g de DHC (0,610 mole), 300 mL d'eau, 42,5 g d'une solution aqueuse d'HCl à 5 moles/L d'HCl (0,18 moles), 10 g de bromure de sodium (0,097 mole) et 2,4 g d'acide phosphomolybdique. 0,1g de DOSS sont ajoutés et l'agitation est mise en route. Lorsque la température du réacteur se stabilise (14°C), on met la pompe d'introduction de la solution de peroxyde d'hydrogène en marche. On introduit, avec un débit constant, 62,2 g d'une solution d'H₂O₂ à 34,5%, soit 0,630 mole, sur une durée de 4h. La température du milieu se stabilise à 16-17°C pendant la coulée de H₂O₂.

Un prélèvement est effectué à 1 heure de coulée afin de vérifier la consommation du peroxyde d'hydrogène introduit, et donne 0,18% de peroxyde résiduel (si H₂O₂ n'était pas consommé on atteindrait environ 1,4 % d'H₂O₂ résiduel en solution).

En fin de coulée de H₂O₂ on laisse le mélange réactionnel sous agitation encore 30 mn. On note alors que le milieu développe une coloration jaune orange typique de la formation de brome. Parallèlement, la température du milieu redescend, indiquant la fin de réaction. Un dosage d'équivalent peroxyde est alors effectué et indique 0,18%, correspondant au brome libre et aux équivalents peroxydiques résiduels.

Le mélange réactionnel est alors filtré sur un filtre en verre fritté de porosité 4, et les eaux-mères obtenues, représentant 402 g, sont réparties en portions de 50 mL dans des flacons de verre afin d'effectuer les tests de stabilité en présence de l'agent réducteur. Les flacons sont agités, puis on laisse reposer ces flacons à température ambiante (15-20°C) pendant plusieurs jours, afin d'observer la formation de dépôt correspondant à la décomposition du système catalytique. Les résultats sont rassemblés dans le Tableau 1.

**Tableau 1**

| ***Réducteur*** | ***Hydrate d'Hydrazine solution à 10%*** | ***Hydrate d'Hydrazine solution à 10%*** | ***NaHSO₃*** |
|---|---|---|---|
| | Ex1-a | Ex1-b | Ex1-c |
| Ajout | 1.15 g | 1.7 g | 0.45 g |
| Aspect 1 jour | Pas de dépôt | Pas de dépôt | Pas de dépôt |
| Aspect 4 jours | Pas de dépôt | Pas de dépôt | Pas de dépôt |
| Aspect 10 jours | Pas de dépôt | Pas de dépôt | Pas de dépôt |

L'AZDN obtenu est lavé deux fois avec 300 g d'eau et on obtient 102,5 g d'AZDN à 9,2% d'eau, soit un rendement de 95%.

La solution de l'exemple 1a conservée pendant 10 jours est soumise à une centrifugation (800 tours/min) qui confirme l'absence de dépôt ou de solides en suspension.

Cet exemple démontre donc l'absence de précipitation des eaux-mères traitées par les agents réducteurs selon l'invention.

### Exemple 2 : Association d'un agent réducteur avec l'heptamolybdate d'ammonium

On répète l'Exemple 1 en employant, à la place de l'acide phosphomolybdique, la même quantité de molybdate d'ammonium et, comme réducteur, NaHSO₃. On note que la solution obtenue selon l'invention ne présente pas de dépôt au bout de 10 jours.

### Exemple 3 (comparatif) : Procédé n'utilisant pas d'agent réducteur

On opère les mêmes réactions que décrit dans les Exemples 1 et 2, sans toutefois utiliser d'agent réducteur. Ces essais sont désignés respectivement par Exemples 3a et 3b.

On observe dans les deux cas que les eaux-mères de filtration sont limpides mais conduisent, au bout de 4 jours ou 10 jours, respectivement, à un dépôt cristallin jaune dans le fond du flacon.

La présence de ce dépôt est confirmée par centrifugation, et démontre l'absence de stabilité des eaux-mères de filtration. Du fait de son insolubilité dans le toluène et de sa couleur, ce dépôt ne peut pas être de l'AZDN et semble plutôt correspondre à des cristaux de composé du molybdène.

### Exemple 4: Procédé comprenant une étape de recyclage des eaux-mères

On utilise le réacteur décrit dans l'Exemple 1. Le bain cryostat est réglé à 18°C et mis en circulation sur le réacteur. On introduit dans le réacteur 116,8 g de DHC (0,610 mole), une solution comprenant 335 mL d'eau, 6,5g d'HCl (0,18 mole), 10g de bromure de sodium (0.097 mole) et 2,4g d'acide phosphomolybdique. On ajoute 0,1 g de DOSS et l'agitation est mise en route. Au bout de 10 à 15 mn, on met la pompe d'introduction de la solution de peroxyde d'hydrogène en marche. On introduit via la pompe péristaltique 62,2 grammes (0,63 mole) d'une solution d'H₂O₂ à 34,5% en H₂O₂ sur une durée de 4h.

Pendant la coulée de H₂O₂, la température se stabilise à une valeur de 22°C environ. Un prélèvement est effectué à différents temps au cours de l'essai afin de vérifier la consommation du peroxyde d'hydrogène introduit. En fin de coulée de l'H₂O₂ on laisse le mélange réactionnel sous agitation pendant 2 heures. On ajoute alors une solution d'hydrate d'hydrazine diluée (10%) pour neutraliser l'excès indiqué par le dosage peroxydique.

Le mélange réactionnel est ensuite filtré sur un filtre en verre fritté de porosité 4 et les eaux-mères sont recueillies séparément. L'AZDN brut obtenu est lavé quatre fois avec 200g d'eau.

350 g des eaux-mères recueillies sont réengagées dans un nouvel essai avec 116,8g de DHC (0,61 mole) et avec un appoint de 3,3 g d'HCl 36% (0,033 mole), 2 g de NaBr (0,019 mole) et 0,48 g d'acide phosphomolybdique, afin de conserver les concentrations initiales en bromure, catalyseur et acide. Trois opérations de recyclages sont ainsi enchaînées de façon consécutive en gardant les mêmes conditions de température, quantité et vitesse de coulée de l'H₂O₂, ainsi que le temps de réaction.

Les résultats sont reportés dans le Tableau 2 suivant.

**Tableau 2**

| ***Ex.*** | ***% massique H₂O₂ résiduel au cours du temps*** | | | | ***Pureté AZDN*** | ***Rendement*** | ***Mo teneur*** |
|---|---|---|---|---|---|---|---|
| | 1h | 2h | 4h | 6h | % molaire (RMN¹H) | g/L (ICP) | g/L (ICP) |
| 4a | 0.42% | 1.04% | 0.17% | 0.18% | 99.7 | 95% | 2.77 |
| 4b | 0.99% | 1.70% | 0.10% | 0.09% | 99.7 | 97% | 2.7 |
| 4c | 0.95% | 1.50% | 0.12% | 0.15% | 99.7 | 96% | 2.65 |
| 4d | - | - | 0.13% | 0.13% | 99.7 | 95% | - |

Cet essai démontre que la réactivité du système se stabilise au bout d'un à deux recyclages. Le rendement et la pureté de l'AZDN obtenu sont par ailleurs excellents.

### Exemple 5 : Procédé comprenant une étape d'adsorption du molybdène contenu dans les eaux-mères

Les eaux-mères, de coloration bleue intense, obtenues à l'exemple 4d, sont soumises à un test d'adsorption du molybdène en présence de charbon actif.

1,0 g ou 0,5 g de charbon actif en poudre (Norit^{®} SX 2 surface BET 900 m²/g, d90 de 110 µm, disponible dans le commerce) sont ajoutés à 100 mL d'eaux-mères obtenues à l'exemple 4d et l'ensemble est maintenu sous agitation à température ambiante. On note au cours du temps la décoloration du milieu en mesurant l'absorbance à la longueur d'onde de 425 nm correspondant à une bande d'absorption des complexes de bleu de molybdène, au moyen d'un appareil HACH^{®} DR 4000 équipé d'une cuve carrée de 10 mm.

On observe que le charbon actif permet une adsorption pratiquement quantitative des espèces molybdène présentes dans les eaux-mères.

### Exemple 6 : Procédé comprenant une étape d'adsorption et de recyclage du molybdène

On reproduit un essai identique à l'exemple 4a. Les eaux-mères sont rassemblées avec les eaux de lavage de l'AZDN et traitées avec 10 g de charbon poudre Norit^{®} SX2 pendant 60 mn sous agitation à température ambiante. Le charbon actif est séparé par filtration puis retraité par 40 mL de solution de soude 2% sous agitation à température ambiante. On rince ensuite le charbon avec encore 60 mL d'eau, et on obtient environ 100 mL de solution contenant le catalyseur molybdène récupéré. Ces 100 mL sont réengagés dans une réaction selon l'exemple 4a en lieu et place des 2,4 g du catalyseur acide phosphomolybdique, et l'on ajuste l'eau ajoutée à 235 mL pour tenir compte de l'eau apportée par la solution de catalyseur molybdène engagée. Au bout de deux heures de coulée de l'H₂O₂, le taux de peroxyde dosé résiduel est de 1,4%, il est de 0,3% à 4h et il est de 0,2% au final (6h). On observe donc une réactivité comparable à celle de l'essai 4b pour lequel on effectuait un recyclage partiel des eaux-mères avec un complément de catalyseur acide phosphomolybdique. Le rendement est de 95%.

### Exemple 7a : Procédé comprenant une étape d'adsorption du molybdène

On utilise le réacteur décrit dans l'Exemple 1. Le bain cryostat est réglé à 8°C et mis en circulation sur le réacteur. On introduit dans le réacteur le DHC (116,8 g soit 0,610 mole), une solution comprenant 335 mL d'eau, 6,5g d'HCl (0,18 mole), 20 g de bromure de sodium (0,097 mole) et 3,7 g de molybdate de sodium dihydraté. On ajoute 0,1 g de DOSS et l'agitation est mise en route. Au bout de 10 à 15 mn, on met la pompe d'introduction de la solution de peroxyde d'hydrogène en marche. On introduit via la pompe péristaltique 62,2 grammes (0,63 mole) d'une solution d'H₂O₂ à 34,5% en H₂O₂ sur 4h. Le suivi de la concentration résiduelle en peroxyde au cours de la coulée de l'H₂O₂ montre que dans ces conditions le peroxyde d'hydrogène est consommé au fur et a mesure de la coulée. En fin de coulée, le mélange réactionnel est laissé sous agitation encore 15 mn, puis 1 g de NaHSO₃ est ajouté afin de neutralise le taux de peroxyde résiduel. Au cours de la réaction on a prélevé dans le réacteur 13 mL de solution à fin d'analyse.

Après filtration de l'AZDN, les eaux-mères qui contiennent 8,3 g/L de molybdène (exprimé en molybdate de sodium dihydraté) sont recueillies et l'AZDN brut obtenu (humidité 25%), contenant encore 34 g d'eau-mère, est lavé avec environ 800 mL d'eau. 295 g d'eaux-mères de filtration sont conservées pour un recyclage à l'Exemple 7b suivant. On mélange le restant des eaux-mères de filtration (104 g) avec les eaux issues du lavage de l'AZDN qui contiennent, par dosage, 0,32 g/L de molybdate dihydraté. On obtient 962 g de solution contenant 1,2 g/L de molybdate de sodium dihydraté. On traite cette solution pendant une heure sous agitation à température ambiante avec 10 grammes de charbon en poudre NORIT^{®} SX2. Cette solution est filtrée. La solution aqueuse filtrée, qui représente l'effluent produit, est dosée à 0,08 g/L (soit 80 ppm) en molybdate de sodium dihydraté.

Cet essai démontre que le traitement sur charbon actif permet de réduire de façon très importante la teneur en molybdène rejetée.

Le charbon actif est récupéré et remis en suspension dans 10 mL de soude 4%, puis filtré et lavé avec 10 mL d'eau. Cette opération est recommencée une fois. On a récupéré ainsi plus de 80% du molybdène de l'effluent.

Les filtrats issus du traitement avec la solution de soude et du lavage à l'eau du charbon actif sont rassemblés et cette solution (40 g) est réutilisée dans l'exemple 7b suivant.

### Exemple 7b : Procédé comprenant une étape de recyclage du molybdène adsorbé

Le bain cryostat de l'Exemple 1 est réglé à 8°C et mis en circulation sur le réacteur. On introduit dans le réacteur le DHC 116,8 g (0,610 mole), 295 g d'eaux-mères de filtration obtenues à l'Exemple 7a, 40 g de la solution de molybdate obtenue en fin de l'exemple 7a et 0,2 g de molybdate de sodium dihydraté afin de compenser les pertes provenant des prélèvements pour analyse et de la perte dans l'effluent après traitement au charbon obtenus à l'Exemple 7a. Un appoint de 6,8 g d'HCl 36% (0,063 mole), 6,4 g de NaBr (0,067 mole) et 10 mL d'eau est effectué afin de retrouver des concentrations en HCl et NaBr comparables à l'essai de l'Exemple 7a. On ajoute 0,1 g de DOSS et l'agitation est mise en route. Au bout de 10 à 15 mn on met la pompe d'introduction de la solution de peroxyde d'hydrogène en marche. On introduit via la pompe péristaltique 62,2 grammes (0,63 mole) d'une solution d'H₂O₂ à 34,5% sur 4h. Le suivi de la concentration résiduelle en peroxyde au cours de la coulée de l'H₂O₂ montre que, dans ces conditions, le peroxyde d'hydrogène est consommé au fur et a mesure de la coulée, comme dans l'Exemple 7a. On obtient après filtration et lavage de l'AZDN un rendement de 97%.

### Exemple 8a : Procédé comprenant une étape de concentration des eaux-mères

Le bain cryostat de l'Exemple 1 est réglé à 15°C et mis en circulation sur le réacteur. On introduit dans le réacteur le DHC (116,8 g soit 0,610 mole), une solution comprenant 335 mL d'eau, 6,5 g d'HCl (0,18 mole), 10 g de bromure de sodium (0,097 mole) et 2,4 g d'heptamolybdate d'ammonium tetrahydraté. On ajoute 0,1 g de DOSS et l'agitation est mise en route. Au bout de 10 à 15 mn, on met la pompe d'introduction de la solution de peroxyde d'hydrogène en marche. On introduit via la pompe péristaltique 62,2 grammes (0,63 mole) d'une solution d'H₂O₂ à 34,5% sur 4h. En fin de coulée, le mélange réactionnel est laissé sous agitation encore 15 mn, puis 2 g de NaHSO₃ sont ajoutés afin de neutraliser le taux de peroxyde résiduel en fin de réaction (0,2%). L'AZDN obtenu est filtré puis lavé et on obtient un rendement de 95%.

360 g d'eaux-mères, issues de la filtration du milieu réactionnel après réaction, et représentant donc 83% de la totalité des eaux-mères théoriques après réaction, sont soumises à une concentration sous un vide de 250 millibars entre 55 et 60°C. On récupère 295 g de solution concentrée, soit une concentration de 20% de la solution, qui correspond pratiquement à l'eau formée par la réaction et celle apportée par la solution de peroxyde d'hydrogène et le DHC humide. Cette solution concentrée contenant le système catalytique peut donc être réemployée (à l'Exemple 8b) telle quelle, en remettant en jeu 83% de la quantité de DHC et d'H₂O₂ initiale de l'essai, sans ajout de catalyseur molybdate, bromure ou acide.

Des analyses de la DCO contenue dans les eaux-mères avant et après concentration montrent que, par ce procédé, on peut diminuer le taux de DCO contenu dans la solution concentrée de façon importante :
Eaux-mères avant concentration : DCO = 8,9g/L
Eaux-mères après concentration de 20%, DCO = 6,7 g/L

Le distillat ne contenant plus de système catalytique (bromure, molybdène) peut être plus facilement rejeté ou traité par incinération.

### Exemple 8b : Procédé comprenant une étape de recyclage des eaux-mères concentrées

Le bain cryostat de l'Exemple 1 est réglé à 15°C et mis en circulation sur le réacteur. On introduit dans le réacteur le DHC (96 g soit 0,50 mole) et 295 g d'eaux-mères concentrées obtenues à l'Exemple 8a. On ajoute 0,1 g de DOSS et l'agitation est mise en route. Au bout de 10 à 15 mn, on met la pompe d'introduction de la solution de peroxyde d'hydrogène en marche. On introduit via la pompe péristaltique 51,5 grammes (0,52 mole) d'une solution d'H₂O₂ à 34,5% sur 4h. Le suivi de la concentration résiduelle en peroxyde au cours de la coulée montre que, dans ces conditions, le peroxyde d'hydrogène est consommé au fur et a mesure de la coulée comme dans l'Exemple 8a. On observe ainsi que le système catalytique conserve sa réactivité. On obtient après filtration et lavage de l'AZDN un rendement de 95%.

### Exemple 9 : Étude de stabilité des eaux-mères concentrées

Le bain cryostat de l'Exemple 1 est réglé à 7°C et mis en circulation sur le réacteur. On introduit dans le réacteur 60 g d'AZDN (0,304 mole), le DHC (89,2 g soit 0,469 mole), une solution comprenant 335 mL d'eau, 6,5 g d'HCl (0,18 mole), 20g de bromure de sodium (0,097 mole) et 3 g d'acide phosphomolybdique. On ajoute 0,1 g de DOSS et l'agitation est mise en route. Au bout de 10 à 15 mn, on met la pompe d'introduction de la solution de peroxyde d'hydrogène en marche. On introduit via la pompe péristaltique 47,6 grammes (0,48 mole) d'une solution d'H₂O₂ à 34,5% sur 4h. Le suivi des peroxydes au cours de la coulée de l'H₂O₂ montre que celle-ci est consommée au fur et à mesure. En fin de coulée le mélange réactionnel est laissé sous agitation encore 15 mn, puis 2 mL de solution HHZ à 10% sont ajoutés afin de neutraliser le taux de peroxyde résiduel (0,1%) en fin de réaction. L'AZDN obtenu est filtré puis lavé et on obtient un rendement de 95%.

Les eaux-mères récupérées sont concentrées sous 250 millibar à une température de 52-58°C jusqu'à éliminer 40 g de solution (concentration à 40%). Une analyse chromatographique est réalisée sur l'acétone qui est l'un des principaux sous-produits contenus dans les eaux-mères.

0,1% d'acétone sont dosés sur les eaux-mères avant concentration. Après concentration à 40%, plus aucune trace d'acétone n'est décelée (moins de 0,05%).

Les eaux-mères ainsi concentrées restent stables, sans apparition de précipité ou dépôt, pendant plus de 4 jours et peuvent être directement réutilisées en réaction.

### Exemple 10 : Procédé comprenant une étape de recyclage d'AZDN

### Exemple 10-1

Dans un réacteur de capacité 1,5 litre muni d'un système d'agitation permettant le brassage d'une suspension, on introduit 126 g d'AZDN (0,78 mole), 631 g d'eau contenant 12 g d'HCl (0,33 mole), 19 g de NaBr (0.18 molle), 4,52 g d'acide phosphomolybdique (teneur en Mo de 50% soit 0,023 mole de molybdène) et 0,1 g de DOSS. Après mise en route de l'agitation et du système de refroidissement par double enveloppe, on attend que la température se stabilise vers 18°C.

On introduit ensuite en continu 78,05 g d'H₂O₂ à 35% (soit 0,80 mole d'H₂O₂) sur une durée de 4 heures. Au cours de la réaction le milieu est maintenu à une température comprise entre 18 et 20° C. On arrête la réaction environ 20 à 30 mn après la fin d'introduction de H₂O₂. La fin de réaction est visible par la formation de brome et est suivie à l'aide d'une sonde rédox Pt (en début d'introduction H₂O₂, le potentiel est d'environ 500 mV, en fin de réaction le potentiel est d'environ 800 mV).

Un dosage des peroxydes résiduels est effectué et 4 mL de solution de HHZ à 10% sont ajoutés pour 0,1% d'équivalent H₂O₂ en solution.

On filtre ensuite en totalité la suspension du réacteur. On obtient 245 g d'AZDN (sec), soit un rendement de 95%. Les eaux-mères de filtration sont conservées pour le recyclage partiel de l'essai suivant.

### Exemples 10-2 à 10-11

Dans le réacteur de l'Exemple 10-1 on introduit 126 g (0.78 mole) de l'AZDN obtenu, 128 g de DHC (0,78 mole), 0,1g de DOSS et 507,5 g d'eaux-mères issues de la filtration du milieu réactionnel lors de l'essai précédent. On ajoute ensuite 148 g d'eau contenant 2,84 g d'HCl, 6 g de NaBr et 1,45 g d'acide phosphomolybdique, afin de maintenir un milieu réactionnel de même composition que celle de l'Exemple 10-1 (compensation des pertes due à la partie non recyclée des eaux-mères, en tenant compte de la dilution due à l'appoint d'eau par les réactifs et la formation d'eau par la réaction).

La réaction est menée ensuite comme dans l'Exemple 10-1, en coulant de façon continue 78,05 g d'H₂O₂ à 35% sur une durée de 4 heures dans le milieu réactionnel maintenu entre 18 et 20°C.

On opère ainsi en recyclant dans l'essai n+1 une partie de l'AZDN et des eaux-mères obtenues à l'essai n.

Une mesure de granulométrie est effectuée à l'aide d'un appareil Masterziser^{®} S. La mesure se fait sur les cristaux humides en utilisant comme dispersant de l'eau et une goutte de tensioactif Igepal^{®} (nonylphénol éthoxylé), après 10 minutes de circulation dans la cellule de mesure. Les résultats sont rassemblés dans le Tableau 3 ci-dessous.

**Tableau 3**

| Ex. | Granulométrie D10(µm) | Granulométrie D50(µm) | Granulométrie D90(µm) |
|---|---|---|---|
| 10-2 | 46 | 110 | 193 |
| 10-3 | 44 | 111 | 200 |
| 10-4 | 49 | 114 | 208 |
| 10-5 | 58 | 126 | 224 |
| 10-6 | 68 | 140 | 241 |
| 10-7 | 65 | 152 | 262 |
| 10-8 | 51 | 148 | 275 |
| 10-9 | 55 | 147 | 280 |
| 10-10 | 55 | 137 | 270 |
| 10-11 | 57 | 138 | 265 |

On voit que la granulométrie moyenne (D50) augmente pour se stabiliser après 5 recyclages à une valeur de 140-150 µm.

La pureté de l'AZDN produit a été contrôlée au cinquième recyclage (Exemple 10-6) et confirme que le procédé permet d'obtenir un produit très pur :

| %molaire relatif | Exemple 10-6 |
|---|---|
| AZDN | 99,8 |
| DHC | 0,12 |
| Méthacrylonitrile | 0,03 |
| Isobutyronitrile | 0,02 |

### Exemple 11 (comparatif) : Procédé de synthèse d'AZDN par oxydation au chlore

### Exemple 11-1

Dans le réacteur de l'Exemple 10 muni d'une arrivée de chlore en fond de réacteur on introduit successivement, 128 g (0,78 mole) de DHC, 126 g d'AZDN, 378 g d'eau, 425 g d'une solution aqueuse contenant 13% d'HCl et 0,1 g de DOSS. L'agitation est mise en service, ainsi que le dispositif de refroidissement (double enveloppe) et on attend que la température soit stabilisée. On introduit ensuite en continu, dans le milieu réactionnel, 57 g de chlore gazeux sur une durée totale de 4 heures. Au cours de la réaction le milieu réactionnel est maintenu à une température comprise entre 18 à 20° C en réglant si besoin la température de la double enveloppe de refroidissement. On arrête la réaction environ 20 à 30 mn après la fin d'introduction de chlore. La fin de réaction peut être suivie à l'aide d'une sonde redox/Pt. L'excès de chlore non consommé conduit à une augmentation du potentiel redox, qui passe de 200 mV à 800 mV en fin d'essai.

On filtre ensuite en totalité la suspension du réacteur pour recueillir séparément l'AZDN et les jus réactionnels. Les jus réactionnels filtrés sont récupérés pour le recyclage à l'essai suivant.

### Exemple 11-2

Dans le réacteur de l'Exemple 10 on introduit 126 g (0,78 mole) de l'AZDN obtenu à l'Exemple 11-1, 128 g de DHC (0,78 mole), 375 g d'eau, 0,1 g de DOSS et 425 g d'eaux-mères issues de la filtration du milieu réactionnel.

La réaction est menée ensuite comme dans l'Exemple 11-1, en introduisant 57 g de chlore gazeux dans le réacteur sur 4 heures et en maintenant le milieu entre 18 et 20°C.

On opère ainsi en recyclant à l'essai n+1 une partie de l'AZDN et des eaux-mères obtenues à l'essai n, comme décrit dans l'Exemple 11-2. On observe que la granulométrie se stabilise au bout de 5 à 8 recyclages vers une valeur moyenne D50 de 100-110 µm, donc plus faible qu'avec le procédé mettant en jeu H₂O₂ comme agent oxydant.

## Revendications

1. Procédé de synthèse d'un composé azo, comprenant les étapes successives consistant à :
a) faire réagir du peroxyde d'hydrogène avec une solution aqueuse d'un composé hydrazo renfermant au moins un acide organique ou inorganique, au moins un bromure de métal alcalin ou d'hydrogène et au moins un composé soluble dans l'eau choisi parmi les sels et acides à base d'un métal catalytique choisi parmi le molybdène et le tungstène, de manière à former une solution renfermant un composé azo,
b) ajouter à la solution obtenue à l'étape (a) au moins un agent réducteur choisi parmi l'hydrazine, le sulfite de sodium, le bisulfite de sodium et leurs mélanges,
c) récupérer tout ou partie du mélange réactionnel ainsi obtenu, le mélange réactionnel non récupéré pouvant être recyclé à l'étape (a),
d) séparer le mélange réactionnel récupéré en une fraction contenant le composé azo et une fraction d'eaux-mères, et
e) éventuellement, laver la fraction contenant le composé azo pour l'isoler.

2. Procédé selon la revendication 1, **caractérisé en ce que** le composé hydrazo est choisi parmi les composés hydrazo symétriques porteurs de fonctions azotées, en particulier de fonctions nitriles ou amines, telles que le 2,2'-hydrazo-bis-isobutyronitrile, le 2,2'-hydrazo-bis-méthylbutyronitrile, le 1,1'-hydrazo-bis-cyclohexanecarbonitrile ou le 2,2'-hydrazo-dicarbonamide, de préférence le 2,2'-hydrazo-bis-isobutyronitrile.

3. Procédé selon l'une des revendications 1 et 2, **caractérisé en ce que** l'acide est un acide inorganique choisi parmi les acides chlorhydrique, sulfurique, bromhydrique, phosphorique et leurs mélanges, de préférence l'acide chlorhydrique.

4. Procédé selon l'une des revendications 1 et 2, **caractérisé en ce que** l'acide est un acide organique choisi parmi les acides formique, acétique et leurs mélanges.

5. Procédé selon l'une quelconque des revendications 1 à 4, **caractérisé en ce que** le bromure est du bromure de sodium.

6. Procédé selon l'une quelconque des revendications 1 à 5, **caractérisé en ce que** le composé soluble dans l'eau est choisi parmi : les sels de métal alcalin ou d'ammonium de molybdène ou de tungstène, l'acide phosphomolybdique et ses sels alcalins ou d'ammonium, l'acide phosphotungstique et ses sels alcalins ou d'ammonium, et les molybdosulfates, et est avantageusement choisi parmi l'acide phosphomolybdique ou un de ses sels alcalins ou d'ammonium.

7. Procédé selon l'une quelconque des revendications 1 à 6, **caractérisé en ce que** la solution aqueuse renferme en outre au moins un agent tensioactif.

8. Procédé selon l'une quelconque des revendications 1 à 7, **caractérisé en ce que** le pH de la solution aqueuse est compris entre 0 et 2.

9. Procédé selon l'une quelconque des revendications 1 à 8, **caractérisé en ce que** le peroxyde d'hydrogène est introduit dans la solution aqueuse à une température de 0 à 40°C, de préférence de 0 à 20°C, pendant une durée allant de 2 à 6 heures.

10. Procédé selon l'une quelconque des revendications 1 à 9, **caractérisé en ce que** l'agent réducteur est l'hydrazine et le composé soluble dans l'eau est l'acide phosphomolybdique.

11. Procédé selon l'une quelconque des revendications 1 à 10, **caractérisé en ce que** le composé azo est séparé par filtration ou centrifugation, de préférence par centrifugation.

12. Procédé selon l'une quelconque des revendications 1 à 11, **caractérisé en ce qu'**il comprend en outre une étape (f) de recyclage de tout ou partie de la fraction d'eaux-mères dans l'étape (a), éventuellement après concentration, et **en ce que** les étapes (a) à (f) sont éventuellement répétées au moins une fois.

13. Procédé selon l'une quelconque des revendications 1 à 12, **caractérisé en ce qu'**il renferme en outre une étape de traitement de tout ou partie de la fraction d'eaux-mères produite dans l'étape (d) et/ou des eaux de lavage produites dans l'étape (e) à l'aide d'un adsorbant, tel que du charbon actif, afin de retenir le métal catalytique.

14. Procédé selon la revendication 13, **caractérisé en ce qu'**il comprend en outre une étape de récupération du métal catalytique sous forme de solution aqueuse, par traitement de l'adsorbant à l'aide d'une solution aqueuse basique.

15. Procédé selon la revendication 14, **caractérisé en ce qu'**il comprend en outre une étape de recyclage dans l'étape (a) de la solution aqueuse de métal catalytique, éventuellement après concentration.

16. Procédé selon l'une quelconque des revendications 1 à 15, **caractérisé en ce que** dans l'étape (c), de 30% à 60% en poids, avantageusement de 45% à 55% en poids, du milieu réactionnel est récupéré.

## Patentansprüche

1. Verfahren zur Synthese einer Azoverbindung, das folgende aufeinanderfolgende Schritte umfasst:
a) Umsetzen von Wasserstoffperoxid mit einer wässrigen Lösung einer Hydrazoverbindung, die mindestens eine organische oder anorganische Säure, mindestens ein Alkalimetallbromid oder Bromwasserstoff und mindestens eine aus Salzen und Säuren auf Basis eines aus Molybdän und Wolfram ausgewählten katalytisch wirksamen Metalls ausgewählte wasserlösliche Verbindung enthält, zur Bildung einer Lösung, die eine Azoverbindung enthält,
b) Zugeben der in Schritt (a) erhaltenen Lösung zu mindestens einem aus Hydrazin, Natriumsulfit, Natriumbisulfit und Mischungen davon ausgewählten Reduktionsmittel,
c) Gewinnen der gesamten so erhaltenen Reaktionsmischung oder eines Teils davon, wobei die nicht gewonnene Reaktionsmischung in Schritt (a) zurückgeführt werden kann,
d) Trennen der gewonnenen Reaktionsmischung in eine die Azoverbindung enthaltende Fraktion und eine Mutterlaugenfraktion und
e) gegebenenfalls Waschen der die Azoverbindung enthaltenden Fraktionen zu ihrer Isolierung.

2. Verfahren nach Anspruch 1, **dadurch gekennzeichnet, dass** die Hydrazoverbindung aus symmetrischen Hydrazoverbindungen mit stickstoffhaltigen Funktionen, insbesondere Nitril- oder Aminfunktionen, wie 2,2'-Hydrazobisisobutyronitril, 2,2'-Hydrazobismethylbutyronitril, 1,1'-Hydrazobis-cyclohexancarbonitril oder 2,2'-Hydrazodicarbon-amid, vorzugsweise 2,2'-Hydrazobisisobutyronitril, ausgewählt wird.

3. Verfahren nach einem der Ansprüche 1 und 2, **dadurch gekennzeichnet, dass** es sich bei der Säure um eine aus Chlorwasserstoffsäure, Schwefelsäure, Bromwasserstoffsäure, Phosphorsäure und Mischungen davon ausgewählte anorganische Säure, vorzugsweise Chlorwasserstoffsäure, handelt.

4. Verfahren nach einem der Ansprüche 1 und 2, **dadurch gekennzeichnet, dass** es sich bei der Säure um eine aus Ameisensäure, Essigsäure und Mischungen davon ausgewählte organische Säure handelt.

5. Verfahren nach einem der Ansprüche 1 bis 4, **dadurch gekennzeichnet, dass** es sich bei dem Bromid um Natriumbromid handelt.

6. Verfahren nach einem der Ansprüche 1 bis 5, **dadurch gekennzeichnet, dass** die wasserlösliche Verbindung aus Alkalimetall- oder Ammoniumsalzen von Molybdän oder Wolfram, Molybdatophosphorsäure und deren Alkalimetall- oder Ammoniumsalzen, Wolframatophosphorsäure und deren Alkalimetall- oder Ammoniumsalzen und Molybdosulfaten und vorteilhafterweise aus Molybdatophosphorsäure und deren Alkalimetall- oder Ammoniumsalzen ausgewählt wird.

7. Verfahren nach einem der Ansprüche 1 bis 6, **dadurch gekennzeichnet, dass** die wässrige Lösung außerdem mindestens ein Tensid enthält.

8. Verfahren nach einem der Ansprüche 1 bis 7, **dadurch gekennzeichnet, dass** der pH-Wert der wässrigen Lösung zwischen 0 und 2 liegt.

9. Verfahren nach einem der Ansprüche 1 bis 8, **dadurch gekennzeichnet, dass** das Wasserstoffperoxid bei einer Temperatur von 0 bis 40°C, vorzugsweise 0 bis 20°C, über einen Zeitraum im Bereich von 2 bis 6 Stunden in die wässrige Lösung eingetragen wird.

10. Verfahren nach einem der Ansprüche 1 bis 9, **dadurch gekennzeichnet, dass** es sich bei dem Reduktionsmittel um Hydrazin und bei der wasserlöslichen Verbindung um Molybdatophosphorsäure handelt.

11. Verfahren nach einem der Ansprüche 1 bis 10, **dadurch gekennzeichnet, dass** die Azoverbindung durch Filtration oder Zentrifugation, vorzugsweise durch Zentrifugation, abgetrennt wird.

12. Verfahren nach einem der Ansprüche 1 bis 11, **dadurch gekennzeichnet, dass** es außerdem einen Schritt (f) des Zurückführens der gesamten Mutterlaugenfraktion oder eines Teils davon in Schritt (a), gegebenenfalls nach Aufkonzentrieren, umfasst und die Schritte (a) bis (f) gegebenenfalls mindestens einmal wiederholt werden.

13. Verfahren nach einem der Ansprüche 1 bis 12, **dadurch gekennzeichnet, dass** es außerdem einen Schritt des Behandelns der gesamten in Schritt (d) anfallenden Mutterlaugenfraktion oder eines Teils davon und/oder der in Schritt (e) anfallenden Waschwässer mit einem Adsorptionsmittel, wie Aktivkohle, zur Zurückhaltung des katalytisch wirksamen Metalls enthält.

14. Verfahren nach Anspruch 13, **dadurch gekennzeichnet, dass** es außerdem einen Schritt des Zurückgewinnens des katalytisch wirksamen Metalls in Form einer wässrigen Lösung durch Behandeln des Adsorptionsmittels mit einer basischen wässrigen Lösung umfasst.

15. Verfahren nach Anspruch 14, **dadurch gekennzeichnet, dass** es außerdem einen Schritt des Zurückführens der wässrigen Lösung des katalytisch wirksamen Metalls in Schritt (a), gegebenenfalls nach Aufkonzentrieren, umfasst.

16. Verfahren nach einem der Ansprüche 1 bis 15, **dadurch gekennzeichnet, dass** in Schritt (c) 30 bis 60 Gew.-%, vorteilhafterweise 45 bis 55 Gew.-%, des Reaktionsmediums gewonnen werden.

## Claims

1. A process for synthesizing an azo compound, comprising the successive steps consisting in:
a) reacting hydrogen peroxide with an aqueous solution of a hydrazo compound containing at least one organic or inorganic acid, at least one alkali metal bromide or hydrogen and at least one water-soluble compound chosen from salts and acids based on a catalytic metal chosen from molybdenum and tungsten, so as to form a solution containing an azo compound,
b) adding the solution obtained in step (a) to at least one reducing agent chosen from hydrazine, sodium sulfite and sodium bisulfite, and mixtures thereof,
c) recovering all or part of the reaction mixture thus obtained, the reaction mixture not recovered being able to be recycled into step (a),
d) separating the recovered reaction mixture into a fraction containing the azo compound and a mother liquor fraction, and
e) optionally, washing the fraction containing the azo compound to isolate it.

2. The process as claimed in claim 1, **characterized in that** the hydrazo compound is chosen from symmetrical hydrazo compounds bearing nitrogenous functions, in particular nitrile or amine functions, such as 2,2'-hydrazobisisobutyronitrile, 2,2'-hydrazobismethylbutyronitrile, 1,1'-hydrazobiscyclohexanecarbonitrile or 2,2'-hydrazodicarbonamide, preferably 2,2'-hydrazobisisobutyronitrile.

3. The process as claimed in either of claims 1 and 2, **characterized in that** the acid is an inorganic acid chosen from hydrochloric, sulfuric, hydrobromic and phosphoric acids, and mixtures thereof, preferably hydrochloric acid.

4. The process as claimed in either of claims 1 and 2, **characterized in that** the acid is an organic acid chosen from formic and acetic acids, and mixtures thereof.

5. The process as claimed in any one of claims 1 to 4, **characterized in that** the bromide is sodium bromide.

6. The process as claimed in any one of claims 1 to 5, **characterized in that** the water-soluble compound is chosen from: alkali metal or ammonium salts of molybdenum or of tungsten, phosphomolybdic acid and alkali metal or ammonium salts thereof, phosphotungstic acid and alkali metal or ammonium salts thereof, and molybdosulfates, and is advantageously chosen from phosphomolybdic acid or an alkali metal or ammonium salt thereof.

7. The process as claimed in any one of claims 1 to 6, **characterized in that** the aqueous solution also contains at least one surfactant.

8. The process as claimed in any one of claims 1 to 7, **characterized in that** the pH of the aqueous solution is between 0 and 2.

9. The process as claimed in any one of claims 1 to 8, **characterized in that** the hydrogen peroxide is introduced into the aqueous solution at a temperature of from 0 to 40°C, preferably from 0 to 20°C, for a period ranging from 2 to 6 hours.

10. The process as claimed in any one of claims 1 to 9, **characterized in that** the reducing agent is hydrazine and the water-soluble compound is phosphomolybdic acid.

11. The process as claimed in any one of claims 1 to 10, **characterized in that** the azo compound is separated out by filtration or centrifugation, preferably by centrifugation.

12. The process as claimed in any one of claims 1 to 11, **characterized in that** it also comprises a step (f) of recycling all or part of the mother liquor fraction in step (a), optionally after concentration, and **in that** steps (a) to (f) are optionally repeated at least once.

13. The process as claimed in any one of claims 1 to 12, **characterized in that** it also includes a step of treating all or part of the mother liquor fraction produced in step (d) and/or of the washing waters produced in step (e) using an adsorbent, such as active charcoal, so as to retain the catalytic metal.

14. The process as claimed in claim 13, **characterized in that** it also comprises a step of recovering the catalytic metal in aqueous solution form, by treating the absorbent using a basic aqueous solution.

15. The process as claimed in claim 14, **characterized in that** it is also comprises a step of recycling into step (a) of the aqueous solution of catalytic metal, optionally after concentration.

16. The process as claimed in any one of claims 1 to 15, **characterized in that**, in step (c) from 30% to 60% by weight and advantageously from 45% to 55% by weight of the reaction medium are recovered.
